# EUROPEAN PATENT APPLICATION

(11) **EP 0 803 728 A1**
(43) Date of publication of application: **29.10.1997**
(21) Application number: 96201096.3
(22) Date of filing: 24.04.1996
(51) Int. Cl.: G01N 27/07, G01N 27/02, G01N 33/02

(54) **Method for measuring the conductivity of fluids**

(71) Applicant: CELSIS INTERNATIONAL PLC, Cambridge CB4 4FX (GB)
(72) Inventor: Niktianov, Stoyan Niktianov, 2623 MG Delft (NL)
(74) Representative: Perry, Robert Edward

(57) **Abstract**

Method for measuring the impedance of fluids, especially fluid food products, which are contained in a closed package, by performing the following steps:
a) positioning a first relatively small electrode on the inner surface of a wall of said package,
b) establishing a conducting connection between said first electrode and a terminal outside the package
c) positioning a second electrode adjacent to the outer surface of a wall of said package,
d) connecting an impedance measuring and analysing circuit to the terminal of the first electrode and to said second electrode
e) measuring the impedance between said two electrodes,
f) comparing the measured impedance characteristics with reference characteristics.

## Description

The invention relates to a method for measuring the conductivity of fluids especially fluid food products.

### STATE OF THE ART

It is known that the growth of microbiological organisms in a fluid, especially a fluid food product, causes a change in the electrical conductivity of the fluid. In other words, contamination of a food product can be checked by measuring the conductivity of the fluid and comparing the measured value with a reference value related to the non-contaminated fluid.

A prior art circuit and method for checking of liquid food, for example of milk, beer, fruit juices or the like, is described in US4626833. According to this method a conductivity measuring probe comprising two electrodes is inserted in the flow path of the fluid to be checked. The probe is connected to an impedance measuring circuit in which a predetermined reference value is subtracted from the measured value whereafter variations in the remaining signal are monitored to check if these variations are within predetermined limits.

Similar methods, especially intended for diagnosing both clinical and subclinical mastitis infections in milk cattle on the ground of the altered pattern in the electrical conductivity of the milk, are described in US5302903 and NL8301231.

The above methods are restricted to applications where a probe comprising two electrodes can be brought in contact with the fluid to be measured for instance by inserting the probe in a conduit through which the fluid is transported. In US4626833 the probe is inserted in the supply line of a filling machine for filling suitable packages with fluid. In US5302903 and NL8301231 the probe is inserted in the milk tube.

### OBJECT OF THE INVENTION

There is a need for a method for measuring the conductivity of a fluid after the fluid is transferred and stored into a suitable package which is thereafter hermetically sealed. Fluid food products are generally packed in carton packages of different shape and dimensions, such as rectangular shaped cartons or cylindrical shaped cartons. Non of the prior art methods is suited for providing data about the sterility of the contents of a package for instance at the time the package leaves a store and is delivered to a customer or a retailer. It is therefor difficult to guarantee the quality and sterility of the packed product unless sample packages are opened or in another way invasive measurements are performed.

An object of the invention is now to provide a method for non-invasive measuring the impedance a packed fluid, especially a packed fluid food product.

### SUMMARY OF THE INVENTION

In agreement with said object the invention provides a method for measuring the conductivity of fluids, especially fluid food products, which are contained in a closed package, by performing the following steps:
a) positioning a first relatively small electrode on the inner surface of a wall of said package,
b) establishing a conducting connection between said first electrode and a terminal outside the package
c) positioning a second electrode adjacent to the outer surface of a wall of said package,
d) connecting an impedance measuring and analysing circuit to the terminal of the first electrode and to said second electrode
e) measuring the impedance between said two electrodes,

Because of the relative small dimension of the first electrode de field lines between both electrodes show a concentration near said first electrode. As a result the fluid near the first electrode provides a relative large contribution to the measured impedance. This effect can be increased by making the second electrode relatively large. The more the field lines are concentrated in the neighbourhood of the first electrode, the more the influence of the dimensions of the package become of less importance because the actual measurement is restricted to a relatively small volume around the first electrode.

As such packages made of layered material are known in various embodiments and are often made of an elongated strip of layered material by bending and folding and adhesively connecting preselected parts. Within the scope of the invention it has distinct advantages if the package is made of a material having a layered structure one of said layers consisting of electrically conductive material.

One of the advantages is that the conductive layer acts as an equipotential plane resulting in a further concentration of field lines near the first electrode. A further rather surprising advantage is that the additional impedance between the first electrode and the conductive layer varies in the same sense as the impedance of the fluid leading to a significant increase in the sensitivity of the measurement. Furthermore the presence of a conductive layer provides the possibility to detect pinholes in the layered structure where possibly leakage may occur. The conductive layer also acts as a screen separating the measurement environment from the outside world.

In some cases, especially where the construction of the package allows easy access to the conductive layer, it can be preferable to use the conductive layer as second electrode. In that case the inventive method is further characterised by
a) establishing a conducting connection between said layer of electrically conducting material and a terminal outside the package
b) using said layer of electrically conducting material as second electrode.

The presence of the conductive layer allows the detection of pin holes in the inner layer(s) of the package or even leakage through the whole package wall. The respective embodiment of the method is according to the invention characterised in that
a) a first measurement is performed at a relatively high frequency providing a first value,
b) a second measurement is performed at a relatively low frequency providing a second value,
c) the ratio of both values is calculated which ratio shows a strong increase in case of pin holes in the inner layer(s) of the package and in the more severe case of leakage of the package.

The presence of the conductive layer allows furthermore the detection of leakage or volume change in another manner. The respective embodiment of the method is according to the invention characterised in that the second electrode is formed as the combination of two electrode bodies positioned adjacent the outer surfaces of opposite walls of the package, and that at least a measurement is performed whereby the impedance measuring and analyzing circuit is connected to said two electrode bodies for determining the impedance of the layer(s) of the package between the conductive layer and the second electrode bodies, the value of said impedance being indicative for leakage and/or volume change.

### FIGURES

The invention will be explained in more detail with reference to the attached drawings.

Figure 1a illustrates schematically a rectangular package with a first electrode attached to the back wall and a second electrode attached to the front wall thereof.

Figure 1b illustrates the equivalent electric circuit of the total measured impedance.

Figure 1c illustrates schematically the field lines between the first and second electrodes.

Figure 2a illustrates schematically a rectangular package made of a layered material, one of the layers being electrically conductive.

Figure 2b illustrates the equivalent electric circuit of the total measured impedance in figure 2a.

Figure 2c illustrates schematically the field lines between the first and second electrodes.

### DETAILED DESCRIPTION

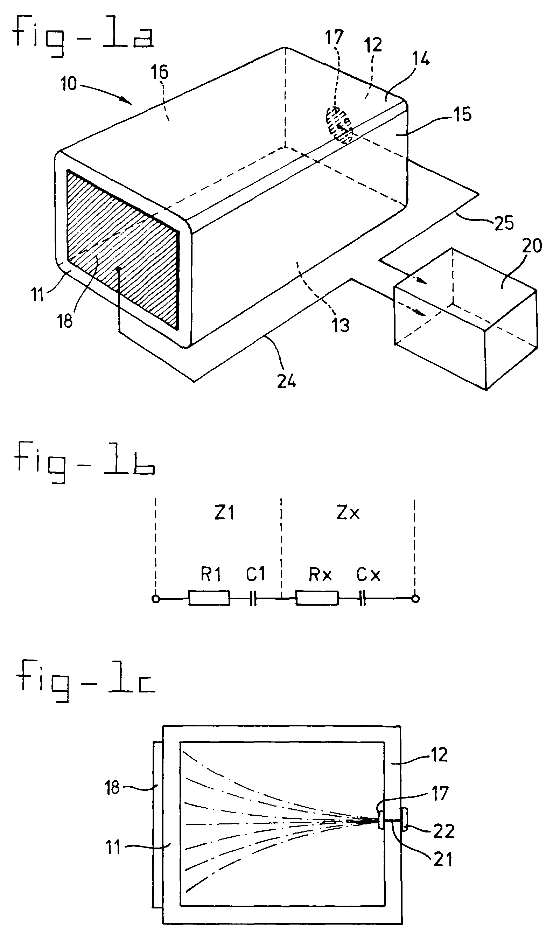

Figure 1a illustrates schematically a rectangular package 10 comprising a front wall 11, a back wall 12, a bottom wall 13, a top wall 14 and two side walls 15 and 16. Figure 1c illustrates a cross section through said package. A first electrode 17 is positioned adjacent to the inner surface of the back wall 15 and a second electrode 18 is positioned adjacent to the outer surface of the front wall 11. The electrode 18 is connected to an impedance measuring and analyzing circuit 20 through a line 24. The electrode 17 is first connected to a terminal 22 on the outer surface of the back wall 12 through a conducting element 21 extending fluid tight through said wall 12. A further line 25 is connected between said terminal 22 and the impedance measuring and analyzing circuit 20. Such circuits are commercially available (for example the instrument model HP4194A manufacturing by Hewlett Packard Inc.) and therefore a detailed description of these instruments is considered superfluous.

Figure 1b illustrates an equivalent electric circuit of the total measured impedance in figure 1a.
Z1 is the impedance between the second electrode 18 and the inner surface of the wall 11,
Zx is the impedance of the fluid food product within the package 10.

Each impedance can be represented by a combination of a resistor and a capacitor as for instance indicated in figure 1b by R1, C1; Rx, Cx.

Figure 1c indicates schematically the extension of the field lines between the relatively small first electrode 17 and the relatively large second electrode 18. As shown the field lines are highly concentrated near the first electrode 17

The system is calibrated by performing one or more measurements with non-contaminated fluid at one or more preselected measuring frequencies. Carefully selecting a frequency is important because the various impedances are more or less frequency dependent. If because of microbiological contamination the value of Zx changes from the non-contaminated reference situation, the analyzing instrument 20 will compare the changed value with the reference value and indicate such variation. In case a rather simple instrument 20 is used the comparison between the "non-contaminated" reference value and the "contaminated" value can of course be performed manually.

In stead of a first electrode adjacent to the inner surface of the wall 12 it is conceivable to attach the first electrode to the outer surface of said wall 12. In that case the terminal 22 and the conducting element 21 can be omitted. In that case the equivalent electric circuit in figure 1B has to be amended by adding another Z1 in series with Zx (assuming that the wall of the package has uniform characteristics all around).

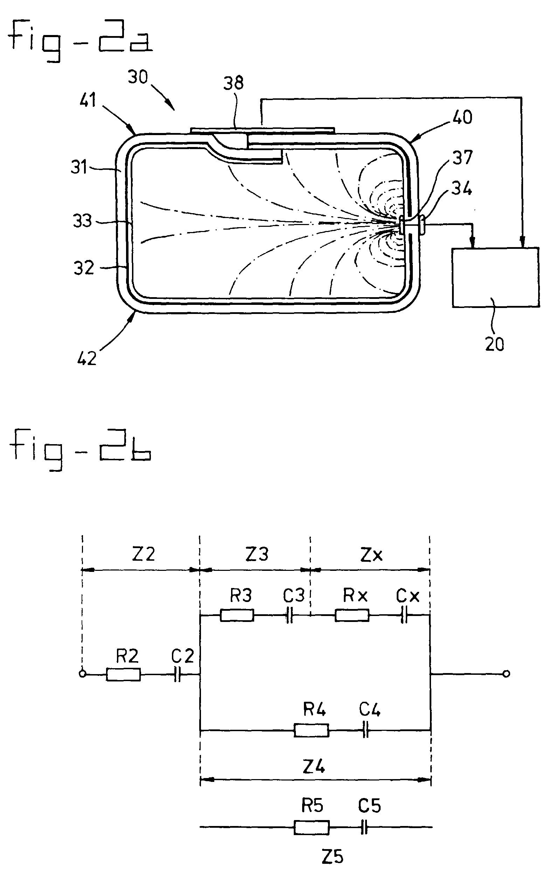

Figure 2a illustrates an application of the method according to the invention with a package 30 of which the wall comprises a layered structure consisting of outer layer(s) 31 of insulating material, an electrically conductive layer 32, preferably of metal such as aluminium, and inner insulating layer(s) 33. The package is made of an elongated strip of layered material by bending and folding and the resulting seam in the top wall is clearly illustrated in figure 2a. The construction of packages of this type is considered to be known to the expert in this field and will therefore not be described in detail.

A first electrode 37 is positioned adjacent the inner surface of the right wall 40 of the package. The second electrode 38 is positioned adjacent the outer surface of the top wall 41. The actual position of the second electrode and also the size thereof are not important because the conductive layer 32 forms an equipotential plane screening the inner volume from the outside world. A second electrode of any size anywhere adjacent to the outer surface of the package will suffice.

Because of the presence of the metal layer 32 the first electrode 37 has to be positioned adjacent to the inner surface of the wall 40. The actual position of the first electrode doesn't matter as long as the first electrode is inside the screen formed by the conductive layer 32. The first electrode 37 is electrically connected to a terminal 34 on the outside surface of wall 40. Of course, measures are taken to avoid contact between the metal layer 32 and the combination of electrode 37, terminal 34, and the inter-connecting lead therebetween. The measuring and analysing circuit 20 is connected on the one hand to the terminal 34 and on the other hand to the second electrode 38.

Figure 2b illustrates the equivalent electric circuit of the configuration in figure 2a.
Z2 is the impedance between the second electrode 38 and the aluminium layer 32,
Z3 is the impedance between the inner surface of layer(s) 33 and the aluminium layer 32,
Zx is the impedance of the fluid food product inside the package,
Z4 is the parasitic impedance between the electrode 37 and the aluminium layer 32.

The presence of the conductive layer 32 has a number of advantages.

The impedance Z2 is a fixed mainly capacitive impedance so that only Z3 and Z4 play a role in the actual measurement of Zx. Z4 is dependent on the dimensions of the first electrode. Surprisingly the impedance Z3 changes as the impedance Zx changes. To explain this the attention is drawn to the field line pattern illustrated in figure 2A This pattern shows a strong concentration of field lines near the first electrode 37. If Rx for some reason decreases from the reference value then the field line distribution will change such that the number of field lines near the first electrode 37 increases. The result thereof is that R3 increases. In other words a change in Rx leads to a change in R3 in the same sense so that in fact the change in Rx is "amplified".

Also this system is calibrated by performing one or more measurements with non-contaminated fluid at one or more preselected measuring frequencies. If because of microbiological contamination the value of Zx changes from the non-contaminated reference situation, the analysing instrument 20 will compare the changed value with the reference value and indicate such variation. In case a rather simple instrument 20 is used the comparison between the "non-contaminated" reference value and the "contaminated" value can of course be performed manually.

Figure 3 illustrates how the resistive part Rx of the measured impedance Zx varies as function of the measuring frequency f used by the circuit 20. Normally an intermediate frequency between f1 and f2 (such as fa) is used. The curve in figure 3 shows a rather linear section between f1 and f2. Deviations from a reference value at a predetermined measuring frequency, for instance caused by an increasing number of microbiological entities, are clearly detectable and measurable.

At frequencies higher then f2 the parasitic capacitor C4 gradually start acting as a short circuit so the use of frequencies higher then f2 is not very practical.

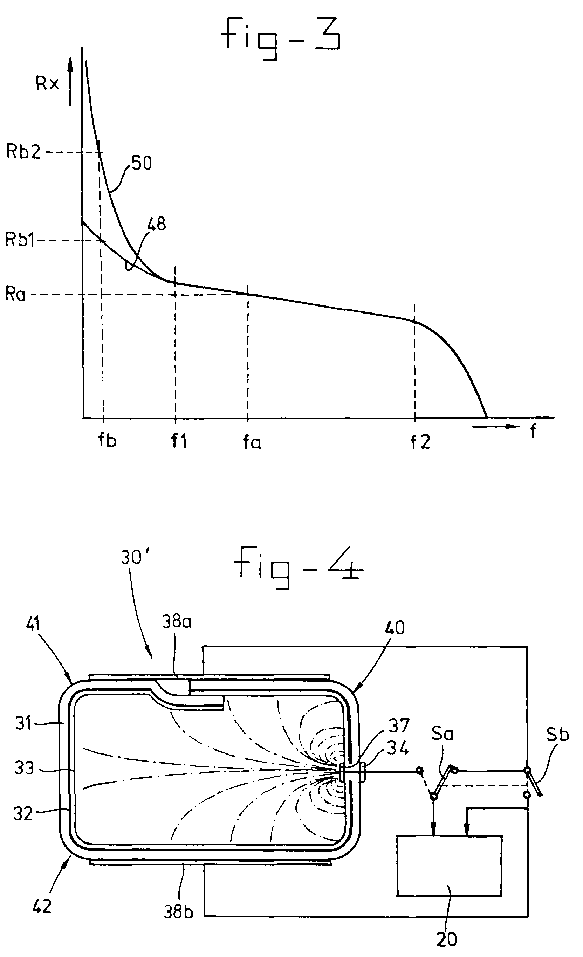

At frequencies lower then f1 the curve of figure 4 shows under normal non-contaminated conditions a slight increase as indicated by 48. In this area of the curve a comparatively strong increase will be observed in case there are pin holes in the inner layer 33 having the effect that there is direct contact between the fluid and the conductive layer 32. If such a situation arises then in fact an additional impedance Z5, represented by R5 and C5 is connected parallel to Z4. At lower frequencies the influence of R5 becomes dominating strong resulting in the sharp increase indicated by 50 in figure 4.

The presence of pin holes or a leak can now be detected by carrying out the following steps:
a) a first measurement is performed at a relatively high frequency (fa) providing a first value (Ra) of the resistive part of the total impedance,
b) a second measurement is performed at a relatively low frequency (fb) providing a second value (Rb1 or Rb2) of the resistive part of the total impedance,
c) the ratio of both values is calculated which ratio shows a strong increase in case of pin holes in the inner layers of the package and in the more severe case of leakage of the package.
   (Zb1/Za which is found in the case of no-leakage is significantly smaller then Zb2/Za which is found in case of leakage).

Figure 4 illustrates a modification 30' of the configuration shown in figure 2A. The differences between both figures are to be found in the embodiment of the second electrode and in the way the various electrodes can be connected to the measuring and analysing circuit 20.

The second electrode 38 is divided in two electrode bodies 38a and 38b, each preferably of the same shape and dimensions. Furthermore said dimensions are preferably selected such that each electrode body covers approximately the whole surface of the respective bottom or top wall of the package 30'.

A double switch Sa,Sb is added to the circuit 20. In the shown condition (full lines) the circuit 20 is connected to the two electrode bodies 38a and 38b. In the other condition (dashed lines) the circuit 20 is connected to the first electrode and to two parallelled electrode bodies 38a, 38b. In this last condition the whole configuration functions exactly as described with reference to figure 2A.

However, in the shown condition a different measurement can be performed. In that case the two electrode bodies 38a and 38b are treated as separate electrodes by means of which the impedance therebetween is measured. Taking into account that the conductive layer 32 forms an equipotential plane in fact the impedance of the outer layer 31 is measured. The measured value changes significant if there is a leak through which the layer becomes wet. In that case the resistance R2 will become smaller and the capacitor C2 will become larger so that the total impedance Z2 will become significantly smaller.

Furthermore this measurement provide an indication of a volume change. If because of contamination gas is formed inside the package resulting in an increase of the total volume and the measurement is carried out always with the same distance between the electrode bodies 38a and 38b, then the increase in volume will result into a higher pressure in the wall of the package. Said higher pressure results into a change in Z2 which is clearly detectable.

It is off course preferably to implement the method in such a manner that all possibilities are taken into account. In other words it is preferred that during a first time period the method described in claim 4 is performed and during a successive second time period the method described in claim 5 is performed, or vice versa.

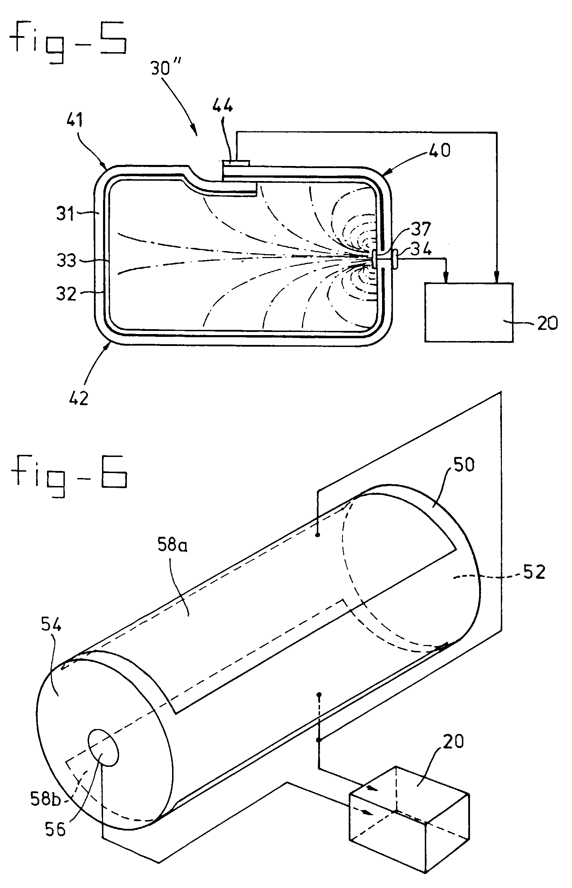

Instead of using a separate second electrode it is also possible to use the conductive layer 32 as second electrode. A corresponding embodiment 30'' is illustrated schematically in figure 5. The conductive layer is in a suitable manner connected to terminal 44 on the surface of the top wall 41, and the terminal 44 is connected to the measuring instrument 20. The equivalent electric circuit is found by making Z2 = 0 in figure 2B.

Figure 6 illustrates another embodiment of a package, in this case a cylindrical package having the cylindrical wall 50, the back end wall 52 and the front end wall 54. A first electrode 56 is positioned adjacent to the front end wall 54 and two electrode bodies 58a and 58b, together forming the second electrode, are positioned adjacent to the cylindrical wall 52 opposite each other and in symmetrical relation to the first electrode 56. Both electrode bodies 58a and 58b are connected in parallel to the conductivity measuring and analysing circuit 20. The circuit 20 is also connected to the first electrode 56.

## Claims

1. Method for measuring the impedance of fluids, especially fluid food products, which are contained in a closed package, by performing the following steps:
a) positioning a first relatively small electrode on the inner surface of a wall of said package,
b) establishing a conducting connection between said first electrode and a terminal outside the package
c) positioning a second electrode adjacent to the outer surface of a wall of said package,
d) connecting an impedance measuring and analysing circuit to the terminal of the first electrode and to said second electrode
e) measuring the impedance between said two electrodes,
f) comparing the measured impedance characteristics with reference characteristics.

2. Method according claim 1, characterised in that the package is made of a material having a layered structure one of said layers consisting of electrically conductive material.

3. Method according to claim 2, characterised by
a) establishing a conducting connection between said layer of electrically conducting material and a terminal outside the package
b) using said layer of electrically conducting material as second electrode.

4. Method according to one of the claims 2 or 3, characterised in that
a) a first measurement is performed at a relatively high frequency providing a first value,
b) a second measurement is performed at a relatively low frequency providing a second value,
c) the ratio of both values is calculated which ratio shows a strong increase in case of pin holes in the inner layers of the package and in the more severe case of leakage of the package.

5. Method according to one of the preceding claims 2-4, characterised in that the second electrode is formed as the combination of two electrode bodies positioned adjacent the outer surfaces of opposite walls of the package, and that at least a measurement is performed whereby the impedance measuring and analysing circuit is connected to said two electrode bodies for determining the impedance of the layer(s) of the package between the conductive layer and the second electrode bodies, the value of said impedance being indicative for leakage and/or volume change.

6. Method according to claims 4 and 5, characterised that during a first time period the method described in claim 4 is performed and during a successive second time period the method described in claim 5 is performed, or vice versa.
